# EUROPEAN PATENT SPECIFICATION

(11) **EP 3 781 216 B1**
(45) Date of publication and mention of the grant of the patent: **14.05.2025**
(21) Application number: 18836383.2
(22) Date of filing: 14.11.2018
(51) Int. Cl.: A61L 2/08, H01J 65/04, H01J 61/16

(54) **EQUIPMENT WITH RADIATION SOURCE FOR FACILITATING KILLING HARMFUL MICROORGANISMS, ESPECIALLY FOR FACILITATION OF CONSERVATION OF FOOD**
AUSRÜSTUNG MIT STRAHLUNGSQUELLE ZUR ERLEICHTERUNG DER ABTÖTUNG VON SCHÄDLICHEN MIKROORGANISMEN, INSBESONDERE ZUR ERLEICHTERUNG DER KONSERVIERUNG VON NAHRUNGSMITTELN
ÉQUIPEMENT AVEC SOURCE DE RAYONNEMENT POUR FACILITER LA DESTRUCTION DE MICROORGANISMES NUISIBLES, EN PARTICULIER POUR FACILITER LA CONSERVATION D'ALIMENTS

(30) Priority: 16.04.2018 HU 1800124
(43) Date of publication of application: 24.02.2021
(73) Proprietor: Vi-At plasmawave UG, 90403 Nürnberg (DE)
(72) Inventor: MÁTÉ, Viktor, 8372 Cserszegtomaj (HU)
(74) Representative: Ronaszéki, Tibor
(86) International application number: PCT/HU2018/000049
(87) International publication number: WO 2019/202343

(56) References cited:
- EP-B1- 1 334 031
- WO-A2-2013/106077
- US-A1- 2006 113 885

## Description

The invention relates to an equipment with radiation source for aiding the destruction of harmful microorganisms, particularly for facilitating the preserving of foodstuffs, said equipment with radiation source comprising two plasma emitters and two operational units, the operational units are connected to a power source, and each of said plasma emitters comprises a quartz glass shell enclosing a vacuum internal space and containing a noble gas charge, and each of said plasma emitter further comprises two external electrical connectors cooperating with said noble gas charge of said internal space.

The subject of the disclosure is an electromagnetic radiation unit, which has closed system plasma radiating glass tubes radiating in the visible range, with a noble gas charge, and is of the indirect electrode type and can be used primarily for food, health, packaging technology, bacteria and virus sterilisation purposes, and also for the removal of living organisms from food and for sterilisation, with good efficiency.

It has been known for a long time that the different microorganisms getting into the human body cause numerous problems, illnesses. It is also well known that the bulk and packaged foods sooner or later go bad, become unusable without preservation, and the cause of all this can also be the microorganisms. There have been numerous solutions worked out for curing the human body, and for disinfection, sterilisation of other user devices. Pasteurization, heat treatment, X-ray treatment, micro wave heat treatment, ionization treatment by UV 2-3 factor, conservation by chemical treatment, treatment by vacuum and noble gas are among them.

Besides the above, those solutions can be listed in such a sterilisation procedure in which radiation is used generated by a plasma emitter for killing the undesirable microorganisms. Such solutions are presented, among others, in the publication documents WO 2011/055113 and WO 2015/039137 and the patent document KR 20150074674.

However, the disadvantage of the known solutions is that they are of complicated structural design, and several such harmonic frequencies appear, besides the frequencies for treatment, emitting by the plasma emitter which are, on the one hand, unnecessary for achieving the desired goal, and, on the other hand, can be harmful for the environment, among them to the living organism as well.

It is another disadvantage of the usual solutions that greater capacity radiation has to be generated in order to achieve that the useful treating frequency reaches the treated material with appropriate intensity, which in the case of given harmonics, regarding the carried performance, may definitely be harmful to health.

In addition to the above the solution with publication number WO 2013/106077 is also known of, which presents equipment adapted for disinfecting enclosed internal areas and objects. The essence of this is that in the interest of a broader scope of use the discharge lamp forming a disinfecting UV radiation source is supplemented with a preferably formed reflector system. In this way by using the equipment the disinfecting UV radiation can be projected over a greater distance and over a more extensive area.

Patent specification registration number EP 1334031 discloses a method with which the treatment of products placed in transparent packaging material may be implemented. For this a diffuser has to be set up in the interior of the package and the treatment may be performed by projecting the light beams originating from the UV radiation source to this diffuser.

The aim of the invention was the elimination of the shortcomings of the known solutions and the creation of an equipment that is capable of the implementation of such a conservation procedure which is capable of sterilization with short residence time radiation at a target frequency, using a small amount of energy, with preferred investment and operational costs, having good performance and efficiency, without additional chemical substances for the conservation of foods, and which does not cause any harmful radiation burden on the environment.

The basis of the invention was the finding that only the sterilisation, preservative techniques operating with a plasma emitter, with only a magnetic field, with an electromagnetic radio wave medium provide the most effective, simplest structural switching technological solutions, which are the least sensitive to external mechanical forces, radio frequency interferences, organic materials to be treated, and their packaging materials, as a dielectric.

As a result of this consideration, the following recognition led to the solution according to the invention: if, as the source of radiation, we place in a specific geometric layout at least two plasma emitting tubes, the structural formation of which is different from the usual one, and the plasma emitting tubes are connected with such an operating unit with which it is possible to create real, precisely modulated, harmonic-free, encircled, regulated sinus frequency radiation, then the created electromagnetic radiation makes it possible to hinder the viability of the pathogens, and fermenting, depleting, putrefactive fungi and moulds, and also of their spores, and of bacteria, viruses and to kill them, even in the inactive state of the microorganisms, even at room temperature, and in this way the task can be solved.

The present disclosure provides an equipment with radiation source for aiding the destruction of harmful microorganisms, particularly for facilitating the preserving of foodstuffs, - which has a radiation source comprising plasma emitters, where each of the plasma emitters has a quartz glass shell, and a noble gas charge located in an evacuated internal space enclosed by the quartz glass shell, and an electric energy source connected to an operational unit, and the operational unit has a signal shape forming sub-assembly and a base signal provider sub-assembly connected to it, - is set up in such a way that the radiation source has plasma emitters adapted for emitting electromagnetic radiation in a frequency range 10 kHz - 1 MHz, furthermore the plasma emitters forming the radiation source each have external electric connections cooperating with the said noble gas charge of the evacuated internal space. furthermore the base signal provider sub-assembly is coupled with an additional signal input sub-assembly, where each of the signal shape forming sub-assemblies which belongs to its own operational unit connected to its own plasma emitter are connected to their own base signal provider sub-assembly via their own base signal input, and are connected to their own additional signal input sub-assembly via their own adjusting input, an output of each of the signal shape forming sub-assemblies is in a signal transmission connection with its wave signal generator sub-assembly via its own filtering-amplification sub-assembly, each of said wave signal generator sub-assemblies has a first output connected to one of the electric connections of its own plasma emitter, a second output connected to another electric connection of its own plasma emitter, and a regulation signal output connected to a feedback regulation input of its own signal shape forming sub-assembly, and the said signal shape forming sub-assemblies operating the individual plasma emitters are harmonised with each other using additional signal input sub-assemblies connected to their adjusting input.

The present invention is defined in the appended claims.

A further feature of the equipment according to the invention may be that the quartz glass shell of the plasma emitter has a thickness of at least 3 mm.

In the case of a different embodiment of the equipment the noble gas charge located in the vacuum internal space of the plasma emitter contains helium-argon or helium-neon.

In the case of another different embodiment of the invention the longitudinal axis of each of the plasma emitters is located in a main plane, and the longitudinal axes of the plasma emitters are at an angle to each other of between 80 to 130°.

In the case of yet another different embodiment of the invention the signal shape forming sub-assembly has an amplitude modulator providing a sine curve frequency, furthermore, the signal shape forming sub-assembly has a programmable microcontroller.

From the point of view of the invention it is preferred if the external electric connections of the plasma emitters are rings each encircling the quartz glass shells from the outside in the vicinity of their ends.

In the case of a further embodiment of the equipment the base signal provider sub-assembly has a variable carrier frequency or a 16 MHz carrier frequency crystal resonator.

The equipment according to the invention has several advantageous features. The most important thing from all of these is that as a result of the specifically designed and arranged plasma emitters and the electrical facilities operating them, the sterilisation of the treated substance, the preservation of the foods can be implemented by the equipment according to the invention with good performance and efficiency, with advantageous investment and operation expenses, low energy consumption, with a short residence time radiating through at a targeted frequency, namely in such a way that the environment does not have any harmful radiation burden.

Further, it is also the advantage of the equipment according to the invention that its construction, constructional solution are simple, its maintaining and possible repair which can be necessary can be carried out at a low costs and work load.

It is a further advantage of it that it is possible to treat by irradiation the raw, non-packed food products in the packaging chain on the conveyor belt, but also the packaged foods which do not contain metallic material, such as bulk packaged, baled, sacked, pallet, unit packed foodstuffs, can also preserved by subsequent irradiation after the packing process. As with the applied electromagnetic radiation, which penetrates the organic and non-metallic substances, the chosen aim can be solved in all respects with maximal efficacy and speed.

It is also a further advantage of the equipment, regarding the invention that its programming makes it possible to preserve the packed, and the not yet packed foods independently of the physical state of the product, in the case of liquids, gels, solid state materials, even in the case of meats, with such intensity and radiation at a frequency according to the pathogens characteristic of the given material.

It is also can be listed among the advantages that the procedure is based on the principles of biophysical resonance, its effect mechanism causes the microorganisms in the "lifeless" foods to be unable to multiply, becoming unviable and destroys them. As the procedure is of not chemical character, it does not apply additives, preservatives, in itself it is not toxic, so it does not transfer any additives into the treated foods, it does not decompose liquid, does not transmit targeted heat to the treated material, does not change the cell-fibre structure in the foods, does not cause change of colour, quality deterioration, does not create new chemical compounds, does not result in a change of volume, evaporation, "loss of smell", or a change of consistency even in the case of irradiation for a longer period of time, in this way it can be used as prevention, with very good efficiency for prevention of toxic effects caused by bacteria in foods.

It is to be considered as an advantage that thanks to the structure of the radiation source, it does not contain mercury, in this way the UV 2-3 radiation factor is very low, there is no ionization in the product, no x-ray radiation, consequently, it does not cause aging to the "green" packaging which breaks down in nature, does not cause harm to it, so it is possible to perform subsequent treatment of the packaged goods which are marketable.

It is an advantage, thanks to the particular operational unit of the equipment, that during the treatment there can be adjusted at the same time several sine wave kinds (interference) of radiating frequencies, the given signal shape and intensity can be adjusted in a programmed way. After the radiation emitted by the equipment goes through the organic materials, liquids, silicates, it causes an effect only to such living microorganisms which are, because of their own vibration, physically sensitive to the adjusted (floated - scanned) sine wave resonance frequencies. The plasma radiation is not static, so the treated food product goods do not get electrically charged.

A further advantage of the solution is that the even distribution, homogeneity as a result of the radio radiation, because of which factors it does not matter what the density, consistency. form, colour, organic component, layering, food type, shape of the substance to be treated is - it is necessary to take into account only the mass - temperature density compensation and packaging, and also the residence time period. But the equipment according to the invention is not capable of effective treatment of substances which are placed into metal shielding.

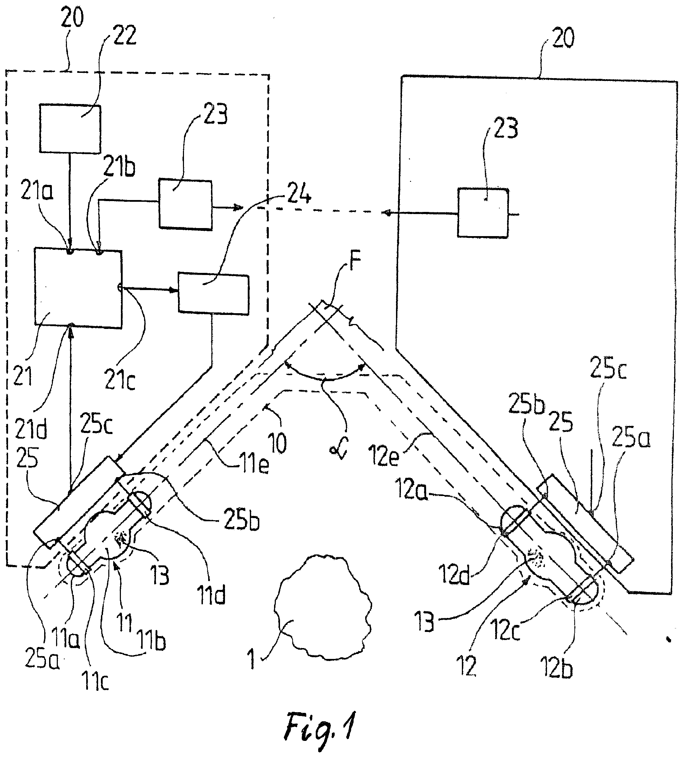

In the following the equipment according to the disclosure is presented in connection with exemplary embodiments, on the basis of a drawing, wherein
Figure 1 shows a schematic block diagram of a version of the equipment.

Figure 1 presents equipment according to the disclosure the radiation source 10 of which contains two identical plasma emitters 11, 12. The plasma emitter 11 and the plasma emitter 12 are arranged as compared to each other so that the longitudinal axis 11e of the plasma emitter 11 and the longitudinal axis 12e of the plasma emitter 12 fall in a single main plane "F", and the longitudinal axis 11e of the plasma emitter 11 and the longitudinal axis 12e of the plasma emitter 12 are at an angle "α" to each other of between 80 and 130°. The material to be treated is located in a space irradiated by the plasma emitter 11 and the plasma emitter 12 and that is free of metal shielding.

The plasma emitter 11 and the plasma emitter 12 of the radiation source 10 are of identical structure; the quartz glass shell 11a of the plasma emitter 11 encloses the internal space 11b, which is provided with a noble gas charge free of mercury.

The quartz glass shell 11a of the plasma emitter 11 has a wall thickness of 3-4 mm and during manufacture is treated with a heated vacuum at a pressure of 10⁻⁹ Pa, then after filing it with the noble gas charge 13 the pressure in the internal space 11b of the plasma emitter is approximately 10⁻⁴ Pa. The noble gas charge 13 may be a helium-argon or even a helium-neon charge, where the ratios of the components may vary. The plasma emitter 11 has an electric connector 11c and an electric connector 11d, which are external electrodes that each have a circular ring located at the two ends of the quartz glass shell 11a of the plasma emitter, which completely encompass the quartz glass shell 11a of the plasma emitter 11. This makes it possible to easily replace the plasma emitter 11. Essentially the rings encircling the quartz glass shell 11a of the plasma emitter 11 transmit the electric energy through the anode electric connector 11c and the cathode electric connector 11d, and the transmitted 1,400-2000 V voltage ignites the noble gas charge 13 located in the internal space 11b encompassed by the quartz glass shell 11a of the plasma emitter 11, and creates the plasma radiation.

As figure 1 illustrates well the plasma emitter 12, similarly to the plasma emitter 11, has a quartz glass shell 12a, which delimits the internal space 12b provided with the noble gas charge 13. The plasma emitter 12 is also provided with a first external electric connector 12c and a second external electric connector 12d, which each have a ring running around the outside of the quartz glass shell 12a, and these support the plasma emitter 12, and conduct the electric current to the noble gas charge 13 in the internal space 12b of the plasma emitter 12.

The plasma emitter 11 of the radiation source 10 is connected to an independent operational unit 20 and the plasma emitter 12 of the radiation source 10 is connected to another independent operational unit 20'. This is preferred because in this way the frequency and other physical parameters of the plasma emitter 11 and the plasma emitter 12 may be independently set. But, naturally, the independent operational units 20 and the independent operational units 20' may be linked to each other and harmonised.

In other respects, the operational unit 20 consists of a signal shape forming sub-assembly 21, a base signal provider sub-assembly 22, an additional signal input sub-assembly 23, as well as a filtering-amplification sub-assembly 24 and a wave signal generator sub-assembly 25, and is connected to an electric energy source which is not indicated in the drawing.

The signal shape forming sub-assembly 21 has a base signal input connector 21a, an adjusting input connector 21b, an output connector 21c and a feedback regulation input connector 21d. The base signal input connector 21a is connected to the base signal provider sub-assembly 22, while the adjusting input connector 21b is connected to the additional signal input sub-assembly 23. The output connector 21c of the signal shape forming sub-assembly 21 is connected to the filtering-amplification sub-assembly 24. The filtering-amplification sub-assembly 24 is connected to the input of the wave signal generator sub-assembly 25. The first output connector 25a of the wave signal generator sub-assembly 25 is electrically connected to the first external electric connector 11c of the plasma emitter 11, while the second output connector 25b of the wave signal generator sub-assembly 25 is electrically connected to the second external electric connector 11d of the plasma emitter 11. The regulation signal output connector 25c of the wave signal generator sub-assembly 25 is connected to the feedback regulation input 21d of the signal shape forming sub-assembly 21.

The task of the signal shape forming sub-assembly 21 is to enable the adjustment of the radiated frequency having physical characteristics that correspond to the given treated substance 1 and/or microorganisms to be destroyed. In the interest of this the signal shape forming sub-assembly 21 is a programmable microcontroller. The base signal provider sub-assembly 22 is preferably a crystal resonator, the task of which is to provide a precise carrier frequency for the signal shape forming sub-assembly 21. The signal shape forming sub-assembly 21 in the given case is a crystal resonator with a carrier frequency of 16 MHz, but, naturally, the carrier frequency of the crystal resonator of the signal shape forming sub-assembly 21 may be different to this.

The additional signal input sub-assembly 23 is responsible for enabling the setting of the precise frequency in the signal shape forming sub-assembly 21 required for the treatment. The additional signal input sub-assembly 23 is responsible for producing and providing this frequency, which is "placed on" the carrier frequency provided by the base signal provider sub-assembly 22.

Using the base signal provider sub-assembly 22 and the additional signal input sub-assembly 23, the signal shape forming sub-assembly 21 is adapted for producing a treatment frequency of between 10 KHz and 1 MHz and a sine signal shaped frequency providing a radiated performance of between 20-70 W. Another task is to be able to adjust the treatment method, therefore it may be decided in the signal shape forming sub-assembly 21 whether the treatment takes place at a single discrete frequency, or the radiation of the treatment frequency range takes place with scanning there-and-back, or possibly with floated sine frequencies. The signal shape forming sub-assembly 21 is also adapted for generating white noise, which is composed of a sequential series of sine signals falling in a well-defined frequency range in the desired tuneable frequency bands.

The filtering-amplification sub-assembly 24 serves for filtering and appropriately amplifying the signal produced in the signal shape forming sub-assembly 21 and its task is to provide the now clean sine signal to the wave signal generator sub-assembly 25. A voltage of between 1,400-2,000 V is supplied between the electrical connector 11c operating as anode and the electrical connector 11d used as the cathode through the first output 25a and the second output 25b of the wave signal generator sub-assembly 25 using the first external electrical connector 11c and the second external electrical connector 11d to ignite the noble gas charge 13 in the internal space 11b enclosed by the quartz glass shell 11a of the plasma emitter 11 and following this to create the plasma radiation implemented at the regulated frequency with a high frequency supply at an operating voltage of between 700-800 V depending on capacity, which radiated vibration waves implement the actual treatment of the treated substance 1, i.e. the destruction of the microorganisms with the targeted frequency.

The operational unit 20' driving the plasma emitter 12 has the same construction and operation, therefore its structure and operation are not detailed here. The only difference between the operational unit 20 belonging to the plasma emitter 11 and the operational unit 20' associated with the plasma emitter 12 is that the first output connector 25a' of the wave signal generator sub-assembly 25' is electrically connected to the first external electrical connector 12c of the plasma emitter 12, while the second output connector 25b' of the wave signal generator sub-assembly 25' is electrically connected to the second external electrical connector 12d of the plasma emitter.

It should be noted here that the additional signal input sub-assembly 23 and the additional signal input sub-assembly 23' may be sources suitable for independent signal feeding, but it is also conceivable that the source signal of the same external signal generator is connected to the additional signal input sub-assemblies 23 of the operational unit 20 driving the plasma emitter 11 and the additional signal input sub-assembly 23' of the operational unit 20' driving the plasma emitter 12.

During the operation of the equipment the desired signal shape, frequency and amplitude, as well as the method of irradiation may be set using the signal shape forming sub-assembly 21 and the required treatment signal may be produced from the vibration modes received from the base signal provider sub-assembly 22 and the additional signal input sub-assembly 23. The created signal passes through the output connector 21c of the signal shape forming sub-assembly 21 into the filtering-amplification sub-assembly 24 where the harmonics lying on the required frequency are filtered and the desired signal amplification takes place. The electrical signal cleaned and amplified to the desired level in this way passes to between the first output connector 25a and the second output connector 25 b of the wave signal generator sub-assembly 25, and starts the ignition and operation of the noble gas charge 13 of the plasma emitter 11.

The same process takes place in the operational unit 20', and finally starts the ignition and operation of the noble gas charge 13 of the plasma emitter 12.

On the basis of the magnitude of the voltage reaching the electrical connector 11c and the electrical connector 11d, and the electrical connector 12c and the electrical connector 12d and its course over time the plasma emitter 11 and the plasma emitter 12 generate the plasma radiation, and so the vibration waves for the irradiation of the treated substance 1.

It should be mentioned here that as a result of their positions and their regulated operation the plasma emitter 11 and the plasma emitter 12 of the radiation source 10 are adapted for modulating the radiation emitted by them as a consequence of the effect of interference as a function of the frequencies emitted by the plasma emitter 11 and the plasma emitter 12, which involves a significant advantage from the point of view of the treated substance 1 and the destruction of the microorganisms presumed to be in it, as discrete frequencies between broad limits may be targeted in space even to individual sections of the treated substance 1.

In the case that the performance produced by the plasma emitter 11 and/or the plasma emitter 12 of the radiation source 10 reaches 70 W, and exceeds this value during operation, then in case of the plasma emitter 11 the wave signal generator sub-assembly 25 of the operational unit 20 detects this and sends a feedback signal to the feedback regulation input connector 21d of the signal shape forming sub-assembly 21. As a result of this the signal shape forming sub-assembly 21 reduces the intensity of the signal sent out through the output connector 21c and thereby down-regulates the magnitude of the signal reaching the wave signal generator sub-assembly 25.

The situation is the same with the plasma emitter 12. The wave signal generator sub-assembly 25' of the operational unit 20' detects when the plasma emitter 12 of the radiation source 10 reaches 70W and sends a feedback signal to the feedback regulation input connector 21d' of the signal shape forming sub-assembly 21'. As a result of this the signal shape forming sub-assembly 21' reduces the intensity of the signal sent out through the output connector 21c' and thereby down-regulates the magnitude of the signal reaching the wave signal generator sub-assembly 25'.

It should be mentioned here that the ideal operation temperature of the treatment is between 30 °C and 45 °C. In the case the treatment is performed at a temperature approaching 45 °C the duration of the irradiation of the foodstuff may be reduced and its efficiency increased, as certain types of undesirable organic life forms, such as the microorganisms in the treated substance 1, are no longer active at the given temperature, and their chemical reaction also changes in a medium at this temperature, they are more sensitive to the irradiated resonance frequency corresponding to them, and perish more quickly.

It is evident that the radiation source 10 of the equipment according to the disclosure may also contain more than two plasma emitters. The number of plasma emitters depends on the extent of the task to be performed, the area to be irradiated, and the size of the area.

The area of application of the equipment according to the disclosure may be the eradication of fermenting, souring, putrefactive fungi, bacteria and viruses in sweet foodstuffs, fruit juices, beverages, fresh goods, furthermore, the prevention of alcohol fermentation, or even the extension of the shelf life of packaged products that have been opened, without the use of additives or preservatives.

The equipment according to the invention may be used to assist the preserving treatment of packaged and unpackaged dry, liquid and gel-based foodstuff products both on conveyors and in packaging and production line systems, for establishing the sterilisation section of the packaging line. In a preferred case the second-level sterilisation, preservation treatment process may be planned for installation on the conveyor belt in the "portioning" phase of the process of finished unpackaged products before packaging.

In the case of subsequent treatment the already packaged foodstuff may be irradiated on the conveyor belt, with equipment installed as an extension to the end of the production line, but it may also be used to implement conventional unit treatment in an "incubator cabin" in the case of packaged goods on pallets.

In the case of liquid products the equipment may be positioned inside the storage tank or in the circulation network. In the case of dark, fibrous substances with a high dry material and colorant content it is preferable to perform the treatment by making the substance flow through the equipment.

**List of references**

| | |
|---|---|
| 1 treated substance | |
| 10 radiation source | 11 plasma emitter |
| | 11a quartz glass shell |
| | 11b internal space |
| | 11c electrical connector |
| | 11d electrical connector |
| | 11e longitudinal axis |
| | 12 plasma emitter |
| | 12a quartz glass shell |
| | 12b internal space |
| | 12c electrical connector |
| | 12d electrical connector |
| | 12e longitudinal axis |
| | 13 noble gas charge |
| 20 operational unit | 21 signal shape forming sub-assembly |
| | 21a base signal input connector |
| | 21b adjusting input connector |
| | 21c output connector |
| | 21d feedback regulation input connector |
| | 22 base signal provider sub-assembly |
| | 23 additional signal input sub-assembly |
| | 24 filtering-amplification sub-assembly |
| | 25 wave signal generator sub-assembly |
| | 25a first output connector |
| | 25b second output connector |
| | 25c regulation signal output connector |
| 20' operational unit | 21' signal shape forming sub-assembly |
| | 21a' base signal input connector |
| | 21b' adjusting input connector |
| | 21c' output connector |
| | 21d' feedback regulation input connector |
| | 22' base signal provider sub-assembly |
| | 23' additional signal input sub-assembly |
| | 24' filtering-amplification sub-assembly |
| | 25' wave signal generator sub-assembly |
| | 25a' first output connector |
| | 25b' second output connector |
| | 25c' regulation signal output connector |
| "F" main plane | |
| "α"angle | |

## Claims

1. Equipment with radiation source for aiding the destruction of harmful microorganisms, particularly for facilitating the preserving of foodstuffs, said equipment with radiation source comprising two plasma emitters (11, 12) and two operational units (20), the operational units (20) are connected to a power source;
wherein each of said plasma emitters (11, 12) comprises a quartz glass shell ( 11 a, 12a) enclosing a vacuum internal space (11 b, 12b) and containing a noble gas charge (13); each of said plasma emitter (11, 12) further comprises two external electrical connectors (11c, 11d, 12c, 12d) cooperating with said noble gas charge (13) of said internal space (11b, 12b);
wherein each of said plasma emitters (11, 12) is adapted for emitting electromagnetic radiation in a frequency range 10 kHz - 1 MHz;
wherein each of said operational unit (20) comprises a signal shape forming sub-assembly (21), a base signal provider sub-assembly (22), an additional signal input sub-assembly (23), a filtering-amplification sub-assembly (24) and a wave signal generator sub-assembly (25);
wherein said signal shape forming sub-assembly (21) is connected to said base signal provider sub-assembly (22) by a base signal input connector (21a), to said additional signal input sub-assembly (23) by an adjusting input connector (21b), to said filtering-amplification sub-assembly (24) by an output connector (21c) and to said wave signal generator sub-assembly (25) by a feedback regulation input connector (21d);
wherein said signal shape forming sub-assembly (21) is in a signal transmission connection with said wave signal generator sub-assembly (25) via said filtering-amplification sub-assembly (24);
wherein each of said wave signal generator sub-assembly (25) has a first output connector (25a) and a second output connector (25b), each output connector (25a, 25b) connected to one of the respective electrical connectors (11c, 12c, 11d, 12d) of said plasma emitter (11, 12);
each of said wave signal generator sub-assembly (25) further comprises a regulation signal output connector (25c) connected to a feedback regulation input connector (21d) of said signal shape forming sub-assembly (21);
wherein the frequencies of said two signal shape forming sub-assemblies (21) operating the individual plasma emitters (11, 12) are configured to be harmonised with each other using said additional signal input sub-assemblies (23).

2. Equipment according to claim 1, **characterised in that** the quartz glass shell (11a, 12a) of the plasma emitter (11, 12) has a thickness of at least 3 mm.

3. Equipment according to claim 1 or 2, **characterised in that** the noble gas charge (13) located in the vacuum internal space (11b, 12b) of the plasma emitter (11, 12) contains helium-argon or helium-neon.

4. Equipment according to any of claims 1 to 3, **characterised in that** the longitudinal axis (11e, 12e) of each of the plasma emitters (11, 12) is located in a main plane (F), and the longitudinal axes (11e, 12e) of the plasma emitters (11, 12) are at an angle (α) to each other of between 80 to 130°.

5. Equipment according to any of claims 1 to 4, **characterised in that** the signal shape forming sub-assembly (21) has an amplitude modulator providing a sine curve frequency.

6. Equipment according to any of claims 1 to 5 **characterised in that** the signal shape forming sub-assembly (21, 21') has a programmable microcontroller.

7. Equipment according to any of claims 1 to 6 **characterised in that** the external electrical connectors (11c, 11d, 12c, 12d) of the plasma emitters (11, 12) are rings each encircling the quartz glass shells (11a, 12a) from the outside at their ends.

8. Equipment according to any of claims 1 to 7 **characterised in that** the base signal provider sub-assembly (22, 22') has a variable carrier frequency crystal resonator.

9. Equipment according to any of claims 1 to 7 **characterised in that** the base signal provider sub-assembly (22, 22') has a 16 MHz carrier frequency crystal resonator.

## Patentansprüche

1. Ausrüstung mit Strahlungsquelle zur Unterstützung der Vernichtung schädlicher Mikroorganismen, insbesondere zur Erleichterung der Konservierung von Nahrungsmitteln, wobei die Ausrüstung mit Strahlungsquelle zwei Plasmaemitter (11, 12) und zwei Betriebseinheiten (20) umfasst, wobei die Betriebseinheiten (20) mit einer Stromquelle verbunden sind;
wobei jeder der Plasmaemitter (11, 12) eine Quarzglashülle (11a, 12a) umfasst, die einen Vakuuminnenraum (11b, 12b) umschließt und eine Edelgasladung (13) enthält; wobei jeder der Plasmaemitter (11, 12) ferner zwei externe elektrische Verbinder (11c, 11d, 12c, 12d) umfasst, die mit der Edelgasladung (13) des Innenraums (11b, 12b) zusammenwirken;
wobei jeder der Plasmaemitter (11, 12) zum Emittieren elektromagnetischer Strahlung in einem Frequenzbereich von 10 kHz - 1 MHz ausgelegt ist;
wobei jede der Betriebseinheiten (20) eine signalformbildende Unterbaugruppe (21), eine Basissignalbereitsteller-Unterbaugruppe (22), eine Zusatzsignaleingangs-Unterbaugruppe (23), eine Filterverstärkungs-Unterbaugruppe (24) und eine Wellensignalgenerator-Unterbaugruppe (25) umfasst;
wobei die signalformbildende Unterbaugruppe (21) mit der Basissignalbereitsteller-Unterbaugruppe (22) durch einen Basissignaleingangsverbinder (21a), mit der Zusatzsignaleingangs-Unterbaugruppe (23) durch einen Einstelleingangsverbinder (21b), mit der Filterverstärkungs-Unterbaugruppe (24) durch einen Ausgangsverbinder (21c) und mit der Wellensignalgenerator-Unterbaugruppe (25) durch einen Rückkopplungsregelungseingangsverbinder (21d) verbunden ist;
wobei die signalformbildende Unterbaugruppe (21) über die Filterverstärkungs-Unterbaugruppe (24) in einer Signalübertragungsverbindung mit der Wellensignalgenerator-Unterbaugruppe (25) steht;
wobei jede der Wellensignalgenerator-Unterbaugruppen (25) einen ersten Ausgangsverbinder (25a) und einen zweiten Ausgangsverbinder (25b) aufweist, wobei jeder Ausgangsverbinder (25a, 25b) mit einem der jeweiligen elektrischen Verbinder (11c, 12c, 11d, 12d) des Plasmaemitters (11, 12) verbunden ist;
wobei jede der Wellensignalgenerator-Unterbaugruppen (25) ferner einen Regelungssignalausgangsverbinder (25c) umfasst, der mit einem Rückkopplungsregelungseingangsverbinder (21d) der signalformbildenden Unterbaugruppe (21) verbunden ist;
wobei die Frequenzen der beiden signalformbildenden Unterbaugruppen (21), die die einzelnen Plasmaemitter (11, 12) betreiben, konfiguriert sind, um unter Verwendung der Zusatzsignaleingangs-Unterbaugruppen (23) miteinander harmonisiert zu werden.

2. Ausrüstung nach Anspruch 1, **dadurch gekennzeichnet, dass** die Quarzglashülle (11a, 12a) des Plasmaemitters (11, 12) eine Dicke von mindestens 3 mm aufweist.

3. Ausrüstung nach Anspruch 1 oder 2, **dadurch gekennzeichnet, dass** die Edelgasladung (13), die sich in dem Vakuuminnenraum (11b, 12b) des Plasmaemitters (11, 12) befindet, Helium-Argon oder Helium-Neon enthält.

4. Ausrüstung nach einem der Ansprüche 1 bis 3, **dadurch gekennzeichnet, dass** die Längsachse (11e, 12e) jedes der Plasmaemitter (11, 12) in einer Hauptebene (F) angeordnet ist und die Längsachsen (11e, 12e) der Plasmaemitter (11, 12) in einem Winkel (α) zueinander zwischen 80 und 130° stehen.

5. Ausrüstung nach einem der Ansprüche 1 bis 4, **dadurch gekennzeichnet, dass** die signalformbildende Unterbaugruppe (21) einen Amplitudenmodulator aufweist, der eine Sinuskurvenfrequenz bereitstellt.

6. Ausrüstung nach einem der Ansprüche 1 bis 5, **dadurch gekennzeichnet, dass** die signalformbildende Unterbaugruppe (21, 21') einen programmierbaren Mikrocontroller aufweist.

7. Ausrüstung nach einem der Ansprüche 1 bis 6, **dadurch gekennzeichnet, dass** die externen elektrischen Verbinder (11c, 11d, 12c, 12d) der Plasmaemitter (11, 12) Ringe sind, die jeweils die Quarzglashüllen (11a, 12a) von außen an ihren Enden umgeben.

8. Ausrüstung nach einem der Ansprüche 1 bis 7, **dadurch gekennzeichnet, dass** die Basissignalbereitsteller-Unterbaugruppe (22, 22') einen Kristallresonator mit variabler Trägerfrequenz aufweist.

9. Ausrüstung nach einem der Ansprüche 1 bis 7, **dadurch gekennzeichnet, dass** die Basissignalbereitsteller-Unterbaugruppe (22, 22') einen 16-MHz-Trägerfrequenz-Kristallresonator aufweist.

## Revendications

1. Équipement avec source de rayonnement pour faciliter la destruction de micro-organismes nocifs, notamment pour faciliter la conservation d'aliments, ledit équipement avec source de rayonnement comprenant deux émetteurs à plasma (11, 12) et deux unités opérationnelles (20), les unités opérationnelles (20) sont connectées à une source d'alimentation;
dans lequel chacun desdits émetteurs à plasma (11, 12) comprend une coque en verre de quartz (11a, 12a) entourant un espace interne sous vide (11b, 12b) et contenant une charge de gaz noble (13); chacun dudit émetteur à plasma (11, 12) comprend en outre deux connecteurs électriques externes (11c, 11d, 12c, 12d) coopérant avec ladite charge de gaz noble (13) dudit espace interne (11b, 12b); dans lequel chacun desdits émetteurs à plasma (11, 12) est adapté pour émettre un rayonnement électromagnétique dans une plage de fréquences de 10 kHz à 1 MHz;
dans lequel chacune de ladite unité opérationnelle (20) comprend un sous-ensemble de mise en forme de signal (21), un sous-ensemble fournisseur de signal de base (22), un sous-ensemble d'entrée de signal supplémentaire (23), un sous-ensemble de filtrage-amplification (24) et un sous-ensemble générateur de signal ondulatoire (25);
dans lequel ledit sous-ensemble de mise en forme de signal (21) est connecté audit sous-ensemble fournisseur de signal de base (22) par un connecteur d'entrée de signal de base (21a), audit sous-ensemble d'entrée de signal supplémentaire (23) par un connecteur d'entrée de réglage (21b), audit sous-ensemble de filtrage-amplification (24) par un connecteur de sortie (21c) et audit sous-ensemble générateur de signal ondulatoire (25) par un connecteur d'entrée de régulation à rétroaction (21d);
dans lequel ledit sous-ensemble de mise en forme de signal (21) est en connexion de transmission de signal avec ledit sous-ensemble générateur de signal ondulatoire (25) par l'intermédiaire dudit sous-ensemble de filtrage-amplification (24);
dans lequel chacun dudit sous-ensemble générateur de signal ondulatoire (25) a un premier connecteur de sortie (25a) et un second connecteur de sortie (25b), chaque connecteur de sortie (25a, 25b) étant connecté à l'un des connecteurs électriques respectifs (11c, 12c, 11d, 12d) dudit émetteur à plasma (11, 12);
chacun dudit sous-ensemble générateur de signal ondulatoire (25) comprend en outre un connecteur de sortie de signal de régulation (25c) connecté à un connecteur d'entrée de régulation à rétroaction (21d) dudit sous-ensemble de mise en forme de signal (21);
dans lequel les fréquences desdits deux sous-ensembles de mise en forme de signal (21) actionnant les émetteurs à plasma individuels (11, 12) sont configurées pour être harmonisées entre elles au moyen desdits sous-ensembles d'entrée de signal supplémentaires (23).

2. Équipement selon la revendication 1, **caractérisé en ce que** la coque en verre de quartz (11a, 12a) de l'émetteur à plasma (11, 12) a une épaisseur d'au moins 3 mm.

3. Équipement selon la revendication 1 ou 2, **caractérisé en ce que** la charge de gaz noble (13) située dans l'espace interne sous vide (11b, 12b) de l'émetteur à plasma (11, 12) contient de l'hélium-argon ou de l'hélium-néon.

4. Équipement selon l'une quelconque des revendications 1 à 3, **caractérisé en ce que** l'axe longitudinal (11e, 12e) de chacun des émetteurs à plasma (11, 12) est situé dans un plan principal (F), et les axes longitudinaux (11e, 12e) des émetteurs à plasma (11, 12) sont à un angle (α) l'un par rapport à l'autre de 80 à 130°.

5. Équipement selon l'une quelconque des revendications 1 à 4, **caractérisé en ce que** le sous-ensemble de mise en forme de signal (21) a un modulateur d'amplitude fournissant une fréquence sinusoïdale.

6. Équipement selon l'une quelconque des revendications 1 à 5, **caractérisé en ce que** le sous-ensemble de mise en forme de signal (21, 21') a un microcontrôleur programmable.

7. Équipement selon l'une quelconque des revendications 1 à 6, **caractérisé en ce que** les connecteurs électriques externes (11c, 11d, 12c, 12d) des émetteurs à plasma (11, 12) sont des anneaux entourant chacun les coques en verre de quartz (11a, 12a) depuis l'extérieur à leurs extrémités.

8. Équipement selon l'une quelconque des revendications 1 à 7, **caractérisé en ce que** le sous-ensemble fournisseur de signal de base (22, 22') a un résonateur à cristal à fréquence porteuse variable.

9. Équipement selon l'une quelconque des revendications 1 à 7, **caractérisé en ce que** le sous-ensemble fournisseur de signal de base (22, 22') a un résonateur à cristal à fréquence porteuse de 16 MHz.
